(19) Europäisches Patentamt
European Patent Office
Office européen des brevets

(11) EP 2 782 554 B1

# (12) EUROPEAN PATENT SPECIFICATION

(45) Date of publication and mention of the grant of the patent:
**12.09.2018 Bulletin 2018/37**

(21) Application number: **12775696.3**

(22) Date of filing: **25.10.2012**

(51) Int Cl.:
***A61K 8/81*** *(2006.01)* ***A61K 8/89*** *(2006.01)*
***A61K 8/891*** *(2006.01)* ***A61Q 15/00*** *(2006.01)*
***A61K 8/02*** *(2006.01)* ***A61K 8/26*** *(2006.01)*
***A61K 8/28*** *(2006.01)* ***A61K 8/37*** *(2006.01)*

(86) International application number:
**PCT/EP2012/071109**

(87) International publication number:
**WO 2013/075906 (30.05.2013 Gazette 2013/22)**

(54) **SOFT SOLID ANTIPERSPIRANT COMPOSITIONS**

SCHWEISSHEMMENDE WEICHE FESTE ZUSAMMENSETZUNGEN

COMPOSITIONS ANTIPERSPIRANTES SOLIDES ET MOLLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.11.2011 US 201161562205 P**

(43) Date of publication of application:
**01.10.2014 Bulletin 2014/40**

(73) Proprietors:
- **Unilever PLC**
**London, Greater London EC4Y 0DY (GB)**
Designated Contracting States:
**CY GB IE MT**
- **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**

(72) Inventors:
- **BIANCHI, James, Michael**
**Trumbull, Connecticut 06611 (US)**
- **BREWSTER, David, Allen**
**Trumbull, Connecticut 06611 (US)**
- **DEGUERVILLE, Elodie, Aurore, Suzanne**
**Leeds**
**Yorkshire LS14 2AR (GB)**
- **LAPINSKI, Bryan Charles**
**Trumbull, Connecticut 06611 (US)**
- **ORMANDY, Kevin, Anthony**
**Leeds**
**Yorkshire LS14 2AR (GB)**
- **KASTRINAKIS, Anestis**
**Leeds**
**Yorkshire LS14 2AR (GB)**

(74) Representative: **McHugh, Paul Edward**
**Unilever PLC**
**Unilever Patent Group**
**Colworth House**
**Sharnbrook**
**Bedford**
**Bedfordshire MK44 1LQ (GB)**

(56) References cited:
**EP-A1- 2 221 039**
**US-A1- 2002 001 572**
**US-A1- 2002 182 159**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 782 554 B1

## Description

Field of the Invention

This invention relates to substantially anhydrous soft solid antiperspirant compositions having improved stability.

Background of the invention

Soft solids are widely used forms of antiperspirant products. The majority of commercially available soft solid products are anhydrous or substantially anhydrous suspensions that comprise antiperspirant active, carrier oil, and structurant. In such products the antiperspirant active commonly comprises astringent aluminum salt, typically astringent aluminum/zirconium salt, suspended in a matrix formed by a combination of carrier oil and wax structurant. In addition to having relatively high carrier oil content as a function of their overall formulation, i.e., levels typically upwards of 45% by weight, compared to the self-supporting harder products known in the art as "solid" sticks, soft solids tend to have a relatively high ratio of carrier oil to wax structurant. As a result, the tendency for oil separation in soft solids tends to be considerably higher than for solid sticks.

In addition to giving rise to product inconsistency that can negatively impact a product's performance profile, especially as regards sensory performance, in the extreme, oil separation may give rise to pack leakage. Oil separation can be exacerbated when the soft solids are subjected to pressure, shear and/or elevated temperature. As a result, manufacturers may impose limitations on transit and storage requirements for soft solid products, for example, products may be shipped by land rather than air freight, while in hotter climates shorter storage or shelf lives may be specified.

Increasing the amount of wax structurant relative to oil often can improve product stability, however, this can give rise to a stiffer composition with a less creamy texture, and may also result in compositions that are perceived as being heavy or greasy. Changes in the choice of oil and structurant components may also give rise to variations in soft solid stability.

EP2221039A1 describes an anhydrous contact antiperspirant composition comprising a particulate antiperspirant active, a dry particulate shear sensitive encapsulated perfume, a liquid carrier comprising at least one water-immiscible oil and optionally a thickener or gallant for the oil or a suspension aid or a propellant.

One aspect of this invention is to improve the "robustness" of a soft solid antiperspirant by reducing the tendency toward oil separation, particularly under conditions of elevated temperature. Another aspect of this invention is to increase soft solid robustness while also maintaining desirable sensory properties. A further aspect of this invention is to achieve such benefits in a composition that includes a relatively high level of carrier oil in relation to both the wax structurant and the total composition.

Summary of the Invention

It has now been found that by selecting particular combinations of wax structurant and oil components and incorporating the components in certain relative amounts, that soft solid compositions meeting one or more aspects of the subject invention are achieved. In one embodiment there is provided an antiperspirant composition comprising:

a) wax structurant comprising, based on the total weight of the wax structurant:

(i) from 50% by weight -to 75% by -weight of paraffin wax;
(ii) from 15% to 50% by weight of hydrocarbon wax comprising a microcrystalline wa other than paraffin wax, the melting point of the hydrocarbon wax other than paraffin wax being at least 70°C and not more than 95°C, preferably not more than 90°C;
(iii) optionally, up to 10% by weight of silicone wax;

b) carrier oil;
c) at least 15% by weight, based on the total weight of the composition, of astringent antiperspirant active; and
d) optionally, silicone elastomer

wherein:

A) the composition is in the form of a substantially anhydrous soft solid;
B) the ratio, by weight, of the wax structurant to the carrier oil is from 0.085:1 to 0.2:1; -and
C) carrier oil is present in an amount of at least 45% by weight, based on the total weight of the composition; and
D) additional wax, if present, does not exceed 20% by weight of the total weight of the wax structurant

In another embodiment there is provided a method of reducing or controlling perspiration which comprises applying the antiperspirant composition of the subject invention to the underarm area at a dose of from 0.1 to 0.6 grams per underarm.

Detailed Description of the Invention

Compared to solid sticks, soft solids have a creamy texture and are relatively soft products with relatively low "hardness" values, which products are easily spread on skin. The "hardness" of a soft solid product refers to the depth, in millimetres, that a cone penetrates into a test specimen under fixed conditions of time, temperature and mass, as determined in accordance with the procedures of ASTM Method D217-48, incorporated herein by reference, using a Petrotest PNR10 Penetrometer (or equivalent), equipped with an ASTMD2884 plunger (Petrotest Cat. #18-0081 or equivalent, weight =47.5g) and a 2.5g aluminum cone, 20° angle with a base diameter of 10mm, wherein hardness values are reported as an average of 6 replicate measurements. In at least one embodiment, the soft solid compositions of the present invention have a hardness value of from 5 to 25mm, more particularly from 10 to 20mm.

The term "anhydrous" as applied to the subject compositions means that no separate aqueous liquid phase is present and that the antiperspirant composition is free of water, exclusive of any bound or complexed water that may be present in the raw materials, such as, for example, any water of hydration in the antiperspirant active. The term "substantially anhydrous" means that based on the total weight thereof, the antiperspirant composition contains less than 2% by weight of added water, exclusive of any bound or complexed water that may be present in the raw materials. In a preferred embodiment, the antiperspirant composition contains less than 1% of added water. In one embodiment of interest, the antiperspirant composition contains less than 0.5% by weight of added water. Bound or complexed water present in the raw materials is not considered to be "added water" as such term is used herein. Unlike emulsions and other multiple phase compositions with separate internal and external phases, the subject compositions are desirably single phase compositions.

Antiperspirant Active

The composition desirably contains a relatively high content of antiperspirant active. Antiperspirant active is incorporated in an amount of at least 15 % by weight, more particularly at least 18% by weight, and, even more particularly, from 20 to 30% of the weight of the composition, based on the total weight thereof. In at least one embodiment of interest the antiperspirant active is present in an amount of from 22 to 27% by weight of the composition.

Antiperspirant actives for use herein are often selected from astringent active salts, including, in particular, aluminum, zirconium and mixed aluminum/zirconium salts, including both inorganic salts, salts with organic anions and complexes. Preferred astringent salts include aluminum, zirconium and aluminum/zirconium halides and halohydrate salts, such as chlorohydrates and activated aluminum chlorohydrates.

Aluminum halohydrates are usually defined by the general formula $Al_2(OH)_xQ_y{\bullet}wH_2O$ in which Q represents chlorine, bromine or iodine, x is variable from 2 to 5 and x+y=6 while $wH_2O$ represents a variable amount of hydration.

Zirconium actives can usually be represented by the empirical general formula: $ZrO(OH)_{2n-nz}B_z{\bullet}wH_20$ in which z is a variable in the range of from 0.9 to 2.0 so that the value 2n-nz is zero or positive, n is the valency of B, and B is selected from the group consisting of chloride, other halide, sulfamate, sulfate and mixtures thereof. Possible hydration to a variable extent is represented by $wH_2O$. In one embodiment B represents chloride and the variable z lies in the range from 1.5 to 1.87. In practice, such zirconium salts are usually not employed by themselves, but as a component of a combined aluminum and zirconium-based antiperspirant.

The above aluminum and zirconium salts may have coordinated and/or bound water in various quantities and/or may be present as polymeric species, mixtures or complexes. In particular, zirconium hydroxy salts often represent a range of salts having various amounts of the hydroxy group. Aluminum zirconium chlorohydrate may be particularly preferred.

Antiperspirant complexes based on the above-mentioned astringent aluminum and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula $CH_2(NH_2)COOH$.

It is highly desirable to employ complexes of a combination of aluminum halohydrates and zirconium chlorohydrates together with amino acids such as glycine, examples of which are disclosed in U.S. Pat. No. 3,792,068 (Luedders et al). Certain of those Al/Zr complexes are commonly called AZG in the literature. AZG actives generally contain aluminum, zirconium and chloride with an Al/Zr ratio in a range from 2 to 10, especially 2 to 6, an Al/Cl ratio from 2.1 to 0.9 and a variable amount of glycine. Actives of this type are available from suppliers that include Summit Reheis. In one preferred embodiment the active is enhanced activity or activated aluminum/zirconium halohydrate, in particular, activated aluminum-zirconium tetrachlorohydrex glycine (AAZG).

Other actives which may be utilized include astringent titanium salts, for example those described in GB 2299506A.

The proportion of solid particulate antiperspirant salt in a suspension composition normally includes the weight of any water of hydration and any complexing agent that may also be present in the solid active.

In one or more embodiments it is desirable that the mean particle size of the antiperspirant salts is within the range of 0.1 to 100 micron with a mean particle size that is often from 3 to 30 microns, more particularly from 5 to 35 microns, and in certain embodiments of interest from 10 to 25 microns. Actives having either larger or smaller mean particle sizes can also be contemplated.

The particulate antiperspirant active may be present in the form of hollow spheres or dense particles (by which is meant particles which are not hollow). To reduce the appearance of visible deposits on the skin to which the composition is applied or on clothing which comes into contact with the composition, it is preferable for the particles to be substantially free from hollows. Hollows can be eliminated by crushing the spheres.

The composition takes the form of a suspension in which antiperspirant active in particulate form is suspended in the matrix formed by the combination of structurant and carrier oil.

Wax Structurant

This term "wax" is applied herein to a variety of materials including mixtures which have similar physical properties, namely that they are solid materials that are firm to brittle hard, malleable at 20°C. and have a melting point typically in the range of 40°C to 100°C., which materials are water-insoluble and remain water-immiscible when heated above their melting points. The term "melting point" in relation to a wax structurant refers to the endothermic peak in the DSC heating curve for the material, obtained following generally accepted DSC measurement techniques.

Waxes are herein classified as "natural" or "synthetic" waxes. As applied to waxes, the term "natural" refers to waxes that are animal, vegetable or mineral in origin, including waxes that are refined or otherwise treated to remove contaminants or purify. The term "synthetic" as applied to waxes refers to waxes that are synthesized from non-wax starting materials or that are produced by the chemical modification of a wax starting material, the later commonly being a subclass of synthetic waxes known as "semi-synthetic waxes".

The wax structurant of the subject compositions comprises paraffin wax, hydrocarbon wax comprising a microcrystalline wax other than paraffin wax and, optionally, silicone wax. Paraffin wax useful herein is a hydrocarbon wax, typically derived from petroleum or petrolatum. In one form, paraffin wax is predominantly a mixture of aliphatic alkanes that are themselves predominantly linear or normal alkanes, commonly as a mixture of C20 to C40 alkanes, which form is commonly referred to as "hydrocarbon paraffin’ or "linear paraffin", notwithstanding that a minor portion of the paraffin wax may be branched. Forms in which branched alkanes predominate are often referred to as isoparaffin. The melting point of the paraffin wax used herein typically is at least 55°C, and normally less than 70°C. Paraffin wax having a melting point in a temperature range of from 55°C to 65°C is of interest in one or more embodiments, with paraffin wax having a melting point in a temperature range of from 60°C to 65°C being of particular interest.

In addition to paraffin wax, the subject compositions include one or more higher melting hydrocarbon waxes comprising a microcrystalline wax other than paraffin wax, From the perspective of processibility and crystallization behavior the melting point of the additional hydrocarbon wax comprising a microcrystalline wax other than paraffin wax is at least 70°C and not more than 95°C., preferably not more than 90°C. Without wishing to be bound by theory, the inclusion of hydrocarbon wax comprising a microcrystalline wax other than paraffin wax having a melting point above that of the paraffin wax has the potential of minimizing settling of the active by virtue of the higher melting point wax crystallizing out of solution during cooling and increasing the viscosity of paraffin wax-containing melt.

Microcrystalline waxes are commonly produced by the de-oiling of petrolatum. Compared to paraffin wax, microcrystalline wax commonly has as a greater proportion of branched alkanes and a larger portion of higher molecular weight alkanes; compared to paraffin wax, microcrystalline is generally a less brittle, more malleable wax.

When employed the polyethylene wax used herein typically has a nominal molecular weight of from 200 to 2000 Daltons more particularly, from 200 to 1000 Daltons, and even more particularly from 300 to 600 Daltons.

In at least one embodiment of interest by weight, the hydrocarbon wax comprising a microcrystalline wax other than paraffin wax, contains microcrystalline wax as the major component, thereof, with hydrocarbon wax containing, at least 60 percent by weight, more particularly at least 75% by weight of microcrystalline wax, being of particular interest. In at least one embodiment the microcrystalline wax accounts for 90% to 100% by weight of the hydrocarbon wax other than paraffin wax. In another embodiment of interest the hydrocarbon wax other than paraffin wax contains, by weight, from 80 to 100% of microcrystalline wax and from 0 to 10% of polyethylene wax. In yet another embodiment of interest the hydrocarbon wax contains, by weight, from 90 to 100% of microcrystalline wax and 0 to 10% of polyethylene wax.

Other waxes contemplated for use herein as wax structurants are one or more silicone wax structurants including, for example, for example, cetyl-, cetearyl-, stearyl-, behenyl-, C16-18-, C20-24-, C24-C28-, and C30-C45 methyl dimethicones such as, for example, for example, stearyl dimethicone, available from Dow Corning as DC-2503 wax, C16-18

alkyl dimethicone available from Momentive Performance Materials as SF-1632, CF40-45 alkyl dimethicone available from Momentive Performance Materials as SF-1642, alkoxysilanes, such as, for example stearoxytrimethyl silane, available from Dow Corning as DC Q5-0158A wax, behenoxydimethicone available from Evonik as Abil® Wax 2440; polyether silanes such as, for example, bis PEG-18- methyl ether dimethyl silane such as, for example, Dow Corning® 2501, and the like, and mixtures thereof.

While additional waxes, that is to say waxes other than the paraffin, hydrocarbon and silicone waxes described above, may be present, the total amount thereof does not exceed 20% by weight and more particularly does not exceed 15% by weight of the total weight of the wax structurant. In at least one embodiment of interest the composition is free of any additional wax. The term "additional wax" includes, but is not limited to organic ester wax, fatty alcohol, fatty acid waxes, triglycerides of saturated fatty acid, 12-hydroxy stearic acid, and the like. In one or more embodiments it is desirable that, the total amount of additional wax does not exceed 1 % by weight, and preferably does not exceed 0.5% by weight, based on the total weight of the antiperspirant composition. In at least one embodiment of interest, if present, the total amount of fiber-forming polymeric structurant, such as, for example, sterols, N-acylamino acid amides, polyhydroxystearic acid, and the like, does not exceed 1% by weight, and preferably does not exceed 0.5% by weight, based on the total weight of the antiperspirant composition; in another embodiment of interest the composition is free of fiber-forming polymeric structurant.

Wax structurant is desirably employed in the subject compositions in amounts up to 12% by weight. The preferred amount of wax structurant of depends, in part, on the amount, if any, of silicone elastomer and/or other non-wax structuring materials present, for example, particulates such as silica or talc. In many compositions wax structurant is present in an amount of from 5 to 10% by weight, more particularly from 6 to 9% by weight.

Carrier Oil

The water-immiscible carrier oil herein comprises a mixture of materials, which are relatively hydrophobic so as to be immiscible in water, which materials are liquid at 20°C up to at least the temperature at which the structurant is dissolved or dispersed therein. Information as to whether a material is or is not water-immiscible is available from numerous literature sources, for example, the CRC Handbook of Chemistry and Physicspublished by CRC Press. For any material where such data is not available in the literature, it can be measured simply by any chemist using conventional techniques.

As used herein the term "volatile" is used to designate a material having a measurable vapor pressure at 25°C. Typically the vapor pressure of a volatile oil lies in a range of at least 1 Pa or preferably at least 10 Pa at 25°C., though generally will be less than 4kPa (30mmHG). A non-volatile oil can be considered to generate a vapor pressure of below 1 Pa at 25°C.

The carrier oil of a soft solid composition is typically a blend of volatile and non-volatile oils. While the non-volatile oil is frequently selected for its masking ability, to impart desirable sensory properties a large portion of the carrier oil is typically comprised of volatile oil. Volatile oil tends to impart a clean, dry feel to the applied composition, as well as to contribute to smooth product application and glide. Additionally, volatile oil aids in fragrance delivery.

The volatile oil component of the subject invention may comprise from to 0% by 100% weight, more particularly from 50 to 95% by weight, even more particularly, from 70 to 85% by weight of the carrier oil. Also contemplated are embodiments where lesser amounts of volatile oil are present or where volatile oil is absent entirely. In one or more embodiments where volatile oil is present, the soft solid composition includes at least one wax structurant having a melting point in excess of the flash point of at least one volatile oil.

Especially desirable as volatile oil is volatile silicone oil. Compared to many other volatile oils, in packaged suspension compositions such as soft solids, a significant amount of the volatile silicone tends to be retained, as opposed to being lost to evaporation. Volatile retention plays an important role in a product delivering equivalent sensory performance over its useful pack life and is also a factor in product stability. Additionally, in soft solids that include silicone elastomer, a component known to provide a sensory and/or thickening benefit, volatile silicone provides a medium in which the silicone elastomer can swell or expand. It is this swelling of the elastomer and the characteristics of the resulting gel that gives rise to its efficacy as a thickening agent and/or sensory enhancer.

Volatile silicone oils suitable for use herein can be linear or cyclic polyorganosiloxanes or mixtures thereof. Preferred cyclic siloxanes include polydimethylsiloxanes and particularly those containing from 3 to 9 silicone atoms and preferably not more than 7 silicone atoms and most preferably from 4 to 6 silicon atoms, otherwise often referred to as cyclomethicones. Preferred linear siloxanes include polydimethylsiloxanes containing from 3 to 9 silicon atoms. The volatile siloxanes normally by themselves exhibit viscosities of below $10^{-5}$ $m^2$/sec (10 centistokes), and particularly above $10^{-7}m^2$/sec (0.1 centistokes), the linear siloxanes normally exhibiting a viscosity of below 5 x $10^{-6}$ $m^2$/sec (5 centistokes). The volatile silicone oils can also comprise branched linear or cyclic siloxanes such as the aforementioned linear or cyclic siloxanes substituted by one or more pendant $-O-Si(CH_3)_3$ groups. In an embodiment of particular interest, the volatile silicone oil comprises cyclomethicone and preferably comprises cyclopentasiloxane and/or cyclopentasiloxane. Volatile silicone oils are commercially available from numerous suppliers including, for example, Dow Corning Corporation.

Non-limiting examples of other volatile oils that may be present in addition to or as replacement of all or part of the volatile silicone oil are volatile hydrocarbons such as, for example, volatile water-immiscible materials comprising a hydrocarbon chain which optionally can further comprise an embedded ether or ester linkage. It is especially desirable that at least 50% by weight, more particularly, at least 60% by weight, even more particularly, at least 70% by weight of the volatile oil is volatile silicone oil.

The non-volatile oil component of the subject invention typically comprises from 0 to 100% by weight, more particularly from 5 to 50% by weight, even more particularly, from 15 to 30% by weight of the carrier oil, The non-volatile oils are preferably liquid at 15°C, with oils having a boiling point of at least 150°C being particularly advantageous.

Non-volatile oils suitable for use herein include one or more silicone oils, ester oils, ether oils, hydrocarbon oils, alcohol oils and the like, and mixtures thereof. Non-volatile silicone oils are of particular interest, particularly when the volatile oil comprises volatile silicone oil and/or when silicone elastomer is present.

Illustrative, non-limiting examples of non-volatile silicone oils suitable for use in the practice of this invention are: polyalkyl siloxanes, polyalkylaryl siloxanes and polyethersiloxane copolymers. These can suitably be selected from dimethicone and dimethicone copolyols. Such oils are available in a range of viscosities. Silicone oils with viscosities of from 1cst to 1000cst are of particular interest in one or more embodiments. Commercially available non-volatile silicone oils include products available from suppliers that include Dow Corning.

Hydrocarbon oils suitable for use herein may be saturated or unsaturated. The non-volatile hydrocarbon oils often contain from 12 to 40, more particularly, from 20 to 40 carbons on average and include mineral oils, hydrogenated polydecene, hydrogenated polyisobutene and the like.

Non-volatile alcohol oils include, for example, branched chain monohydric alcohols containing from 12 to 40 carbon atoms, and often from 14 to 30 carbon atoms such as, for example, isostearyl alcohol.

Among the suitable ester oils are aliphatic esters, aromatic esters (which term as used in the instant specification and claims includes mixed aromatic/aliphatic ester oils), and triglyceride oils. Suitable aliphatic esters are esters that contain at least one long chain alkyl group such as esters derived from $C_1$ to $C_{20}$ alkanols esterified with a $C_6$ to $C_{22}$ alkanoic acid or $C_6$ to $C_{10}$ alkanedioic acid. Among the suitable aromatic esters are $C_8$-$C_{18}$ alkyl benzoates or mixtures thereof including, in particular, $C_{12}$-$C_{15}$ alkyl benzoates. Many suitable aromatic esters are available under the trademark Finsolv. Other aromatic esters which can be contemplated for use herein comprise double aromatic inclusion. Preferred double aromatic esters comprise a linear or branched alkyl chain, e.g. from 1 to 3 carbons, interposed between ester and/or ether substituted phenyl groups.

Among the triglyceride oils suitable for use herein are natural oils derived from plants. The natural oils desirably comprise one or more triglycerides of oleic acid, linoleic acid, linolenic acid or ricinoleic acid. Various isomers of such acids often have common names, including linolenelaidic acid, trans 7-octadecenoic acid, parinaric acid, pinolenic acid punicic acid, petroselenic acid and stearidonic acid. It is especially desirable to employ glycerides derived from oleic acid, linoleic acid or petroselenic acid, or a mixture containing one or more of them.

Natural oils containing one or more of such triglycerides include, for example, coriander seed oil for derivatives of petroselinic acid, impatiens balsimina seed oil, parinarium laurinarium kernel fat or sabastiana brasilinensis seed oil for derivatives of cis-parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil, olive oil or safflower oil for derivatives of oleic acid, often together with linoleic acids. Other suitable oils are obtainable from hemp, which can be processed to derive stearadonic acid derivatives and maize corn oil. A natural oil that by virtue of its characteristics and availability is of particular interest comprises sunflower oil.

Ether oils suitable for use herein comprise liquid aliphatic ethers, including, for example alkyl ethers of polypropylene glycol (PPG), the alkyl group comprising from 2 to 6, and especially 4 carbon atoms and the PPG moiety comprising from 10 to 20 and particularly 14 to 18 propylene glycol units. One preferred ether oil bears the INCI name PPG14-butyl ether.

In one preferred embodiment the non-volatile oil comprises silicone oil in an amount of at least 45% by weight, more particularly from 50 to 60% by weight, based total weight of non-volatile oil present in the composition.

In one or more embodiments contemplated by this invention at least a portion of the non-volatile oil comprises one or more oils capable of carrying change. Representative of such oils are ether oils, in particular alkoxylated alcohols, for example, liquid aliphatic ethers derived from a polyglycol especially from polypropylene glycol (PPG) containing a least 3 mers, such as 3 to 20, with a monohydric alcohol, preferably a C3 to C20 monohydric alcohol. As the molecular weight of the PPG increases, the chain length of the monohydric alcohol can decrease. For example, suitable ether oils can vary between a low molecular weight PPG with a long chain fatty alcohol, such as PPG-3 myristyl ether and lower alkyl ethers of a higher molecular weight PPG, such as the ether named PPG-14 butyl ether in the CFTA Handbook.

Silicone Elastomers

The silicone elastomers impart a silky feel to the compositions of the invention and also contribute to reducing syneresis of the final product. Silicone elastomers for use herein include cross-linked polydimethyl or polymonomethyl siloxanes optionally having end groups such as hydroxyl or methyl. Such elastomers are commercially available from numerous sources and can be readily made using conventional techniques well known to those skilled in the art.

Preferred silicone elastomers for use in the invention are cross-linked polydiorganosiloxanes, preferably derived from suitable combinations of $R_3SiO_{0.5}$ units and $R_2SiO$ units where each R independently represents an alkyl, alkenyl (e.g. vinyl), alkaryl, aralkyl, or aryl (e.g. phenyl) group. R is most preferably methyl. Such elastomers are described, for example, in U.S. Pat. No. 5,654,362. Among the cross-linkers used in the preparation of such elastomers are: an alpha, omega aliphatic diene cross-linker of the formula:

$$CH_2{=}CH(CH_2)_xCH{=}CH_2$$

where x ranges from 1-20.

Included among such elastomers is DC9040 from Dow Corning.

Other elastomers suitable for use herein include silicone/urethane copolymers having cross-linkers of the formula:

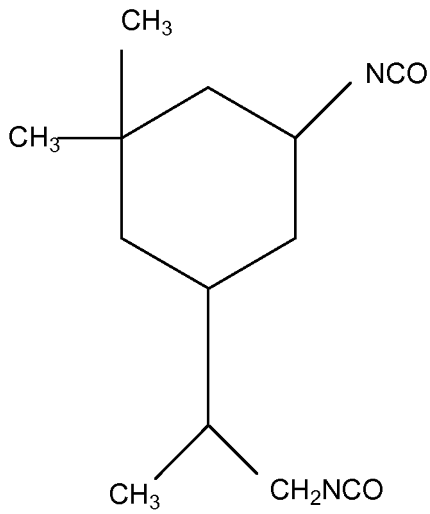

Among the suitable silicone-urethane copolymers is Polyderm PP I-SI-100 from Alzo Incorporated, Matawan, N.J.

Another preferred elastomer is an elastomeric resinous material which is a silicone polymer having a backbone with the following structure:
$R_3SiO(R'_2SiO)_m(R''R'''SiO)_nSiR_3$, where m is 1-250, n is 0-250, R, R', R" are alkyl groups containing 1-6 carbon atoms, and R'" is $CH_2{=}CHCH_2O(CH_2CH_2O)_x(CH(CH_3)CH_2O)_yH$ and x+y is less than or equal to thirty; wherein the polymer backbone is crosslinked with one or more of the following compounds: an alpha-omega diene whose structure is $CH_2{=}CH(CH_2)_zCH{=}CH_2$; an alpha-omega diyne whose structure is $CH{\equiv}C(CH_2)_zC{\equiv}CH$, and an alpha-omega ene-yne whose structure is $CH_2{=}CH(CH_2)_zC{\equiv}CH$, where z ranges from one to twenty.

Yet other preferred elastomers are cross-linked ethoxylated silicone gels such as are available from Dow Corning under the designation DC 9010.

The degree of cross-linking of the silicone elastomers is suitably from about 0.05% to about 35%, preferably being in the range of about 0.15% to about 7%, more preferably from about 0.2 to about 2%.

When present, elastomer is typically included in amounts of from 0.001 to 2% by weight or greater, more particularly, from 0.01 to 1% by weight, based on the total weight of the composition. In one or more embodiments, compositions that contain 0.5% by weight or more of silicone elastomer are of particular interest.

Optional Ingredients

The compositions of this invention may include one or more non-wax rheology modifiers which add thixotropic body or aid in controlling syneresis. Such materials may also assist in processing of the composition while it is in molten

form before being filled into molds. Non-limiting examples of such rheology modifiers include, for example, aluminum stearate, stearamide MEA, silica, in particular, finely divided silica such as fumed or precipitated silica, talc, and mixtures thereof. Silica is among the preferred rheological additives in one or more embodiments.

When present, rheology modifiers are desirably included in compositions of the invention in amounts, based on the total weight of the composition, of up to 4.0% by weight, with amounts of from of from 0.05 to 2.0% by weight, more particularly from 0.1 to 1.5% by weight being of interest in one or more embodiments.

Other ingredients, conventional in the art of soft solid antiperspirant compositions may be included in the compositions of the present invention. Optional ingredients include wash-off agents, often present in the subject compositions an amount of at least 0.05% by weight, and advantageously at least 0.25% by weight up to 5% by weight to assist in the removal of the composition from skin or clothing. When present, the wash-off agent is often present in an amount up to 1%. Such wash-off agents are typically nonionic surfactants such as esters or ethers containing both a Csto $C_{22}$ alkyl moiety and a hydrophilic moiety which can comprise a polyoxyalkylene group (POE or POP) and/or a polyol, e.g., glycerol or sorbitol.

Fragrance is another common optional component. For purposes of this invention, unless otherwise indicated, fragrance is considered as a separate component from the carrier oil, and the amount thereof is not included as the part of the amount of carrier oil permitted in the subject compositions. The total amount of fragrance (inclusive of all material present as part of fragrance encapsulate) is often from 0.001 to 5 wt.%, based on the total weight of the composition In one embodiment, fragrance is desirably employed at a level of from 0.05 to 4wt.%, more particularly from 0.1 to 3.5 wt%, based on the total weight of the composition. Encapsulated fragrance may be formulated as shear or moisture sensitive materials.

Non-limiting examples of other optional ingredients are drying agents, such as talc or aluminum starch octenylsuccinic, skin benefit agents such as allantoin, vitamins or lipids; colors; preservatives; skin cooling agents such as menthol and menthol derivatives; skin feel improvers such as finely divided high melting point polyethylene, micro-fine aluminum oxide powder and/or a particulate polymethylmethacrylate such as Ganzpearl® GMX-0810 from Ganz Chemical.

The amount of such optional adjuncts should not negatively impact the total solid content desired in the subject compositions. When present, the total amount of such optional ingredients typically does not exceed 10% by weight of the composition and often does not exceed 5% by weight of the composition.

If desired the composition can comprise a supplementary deodorant active, i.e., an active other than the antiperspirant salt. Suitable supplementary deodorant actives can comprise deodorant effective concentrations of deoperfumes, and/or microbicides, including particularly bactericides, such as chlorinated aromatics, including biguanide derivatives, of which materials known as triclosan (Irgasan® DP300 from Ciba Specialty Chemicals), tricloban and chlorhexidine warrant specific mention. Supplementary deodorant actives are commonly employed at a concentration of from 0.1 to 5% by weight and often up to 1% by weight of the composition.

The compositions may be employed in dispensers suitable for use with soft solid compositions, preferably one with a plurality of openings through which the composition is dispensed.

Compositions of the instant invention can be prepared by a conventional process in which the wax structurant is melted and dissolved or dispersed in the presence of non-volatile components of the carrier oil and cooled to a safe handling temperature and mixed with antiperspirant active, other particulates, if present, and volatile composition components, the silicone elastomer, if present, typically being incorporated with the volatile oil component after being allowed to swell in same. While still mobile, the resulting mixture is introduced to the product chamber of the container from which it will be dispensed and thereafter is cooled or allowed to cool to below its normal setting temperature, thereby forming a soft solid which can be caused to flow by the application of gentle pressure.

Except in the operating and comparative examples, or where otherwise explicitly indicated, all numbers in this description indicating amounts or ratios of materials, conditions of reaction; physical properties of materials and/or use; dimensions and dimension ratios, are to be understood as modified by the word "about".

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above. It should be noted that in specifying any range of concentration or amount, any particular upper concentration or amount can be associated with any particular lower concentration or amount. Reported ranges are inclusive of their endpoints.

All parts, percentages, ratios, and proportions referred to in the subject specification and in the appended claims are by weight unless otherwise indicated.

The following Examples will more fully illustrate the embodiments of this invention. The examples are not intended to limit the scope of the invention in any manner.

As described in the Examples that follow, composition stability was evaluated by centrifuge testing in accordance with the following procedure.

Centrifuge Test

Centrifuge testing is carried out using a centrifuge equipped with a swing bucket rotor (Beckman- Coulter Ultracentrifuge Model L 735 or equivalent). Testing is carried out using room temperature (25°C) samples obtained from a finished, unused package (the top and center stick, if present, having been removed) or samples filled directly into an empty plastic cylinder (3 cm in diameter and 7 cm deep). To obtain the samples, a preweighed 50 ml centrifuge tube (25mm x 89 mm) is pressed, by hand, straight down into the middle of the soft solid with minimum disruption to the sample. Once pressed into the sample, the tube is given a 90° rotation then extracted. The tube should be nearly full, and the sample size 35 g ± 5g. One sample is taken per pack or tube, and two samples are obtained for each product being evaluated. The weight of each balanced sample-containing tube is measured. The samples are centrifuged at 25°C for 5 minutes at a speed of 2000rmp (720 x g). Any oil that has separated from the sample is pipetted off and discarded, and the sample-containing tubes are reweighed. The percentage of oil separation (alternatively referred to as the Oil Loss Value) is calculated as follows.

$$\%\ \text{oil separation} = \frac{W_2\text{-}W_T}{W_1\text{-}Wt}\ X100$$

where:

$W_1$= the initial weight of the tube with the sample
$W_2$= the weight of the tube with the oil removed
$W_T$ =the weight of the tube empty.

The results are reported as an average of the two samples.

In a variation of this procedure, after being filled and weighed the centrifuge tubes are placed in an oven and maintained at a selected temperature (45°C or 50°C) for a period of 30 minutes, the tubes are then removed from the oven and centrifuged as described above (5 minutes at 2000 rpm (720 x g)), after which they are returned to the oven for an additional 30 minutes, then removed from the oven and centrifuged a second time (5 minutes at 2000 rmp (720 x g)). After being centrifuged for the second time any oil that has separated from the sample is piped off and discarded and the sample-containing tubes are reweighed and the percentage of oil separation is calculated as described above.

Of particular interest are compositions that have an Oil Loss Value of <1%, more particularly ≤ 0.2% at 45°C. Compositions that have an Oil Loss Value of <1 % at 50°C are of particular interest in one or more embodiments.

Examples

1st and 2nd batches of compositions having the formulations described in Tables 1 to 3 were prepared and introduced to soft solid dispensers having a plurality of dispensing openings and suitable for the application of a target dose of 0.4g of product per underarm.

Following the penetrometer and centrifuge procedures described above the 1st batch of compositions was evaluated for hardness and room temperature (25°C) stability, and the 2nd batch of compositions was evaluated for elevated temperature stability (45°C and 50°C).

Results of hardness and stability testing are reported in the tables that follow.

**Table 1**

| **Ingredient (% by weight)** | Example 1 | Example 2 | Example 3 | Example 4 | Example 5 |
|---|---|---|---|---|---|
| Cyclopentasiloxane | balance to 100 | Balance to 100 | Balance to 100 | balance to 100 | Balance to 100 |
| Paraffin Wax (MP 60-62°C) | 3.5 | 3.5 | 3.5 | 3.5 | 3.5 |
| Microcrystalline Wax (MP 80/87°C) | 3.5 | 3.5 | 3.5 | 3.5 | 3.5 |
| Dimethicone (350cst) | | 5.0 | | 5.0 | 10 |
| PPG-14 Butyl Ether | 10.0 | 5.0 | | | |
| C12-18 Alkyl Benzoate | | | 10.0 | 5.0 | |
| Silicone Elastomer* | 4..0 | 4.0 | 4.0 | 4.0 | 4.0 |

(continued)

| Ingredient (% by weight) | Example 1 | Example 2 | Example 3 | Example 4 | Example 5 |
|---|---|---|---|---|---|
| Butylated Hydroxy Toluene (BHT) | 0.05 | 0.05 | 0.05 | 0.05 | 0.05 |
| Aluminum Zirconium Tetrachlorohydrex Glycine | 26.3 | 26.3 | 26.3 | 26.3 | 26.3 |
| Fumed Silica | 1.0 | 1.0 | 1.0 | 1.0 | 1.0 |
| Sunflower oil | 0.5 | 0.5 | 0.5 | 0.5 | 0.5 |
| Fragrance | 0.8 | 0.8 | 0.8 | 0.8 | 0.8 |
| | | | | | |
| **Hardness** (mm) | 19.5 | 13.2 | 24.1 | 16.5 | 16.8 |
| **Centrifuge Results** | | | | | |
| % Oil Separation 25°C | 0.3 | <0.1 | 0.1 | 0.1 | <0.1 |
| % Oil Separation 45°C | None | None | 0.1 | <0.1 | 0.1 |
| % Oil Separation 50°C | 5.6 | 0.4 | 18.4 | 0.8 | 2.3 |
| * DC 9040 Silicone Elastomer from Dow Corning, a mixture of silicone elastomer in cyclomethicone. | | | | | |

**Table 2**

| Ingredient (% by weight) | C1 | C2 | C3 | C4 |
|---|---|---|---|---|
| Cyclopentasiloxane | Balance to 100 | Balance to 100 | Balance to 100 | Balance to 100 |
| Paraffin Wax (MP 60-62°C) | 3.5 | 3.5 | | 3.5 |
| Microcrystalline Wax (MP 80/87°C) | | | 3.5 | |
| Polyethylene 400 (MP 84-86 °C) | | | | |
| Petrolatum (MP 58-64 °C) | | | | |
| Syncrowax® HGL-C C18-36 Acid Triglyceride (MP 70 °C) | | 3.5 | 3.5 | |
| Castor Wax (MP 84 °C) | 3.5 | | | |
| Tribehenin (MP 83°C) | | | | 3.5 |
| Stearyl Alcohol (MP 60°C) | | | | |
| 12-Hydroxystearic acid (MP 72-77°C) | | | | |
| Dimethicone (350 cst) | 10.0 | 10.0 | 10.0 | 10.0 |
| Silicone Elastomer* | 4.0 | 4.0 | 4.0 | 4.0 |
| Butylated Hydroxy Toluene (BHT) | 0.05 | 0.05 | 0.05 | 0.05 |
| Aluminum Zirconium Tetrachlorohydrex Glycine | 26.3 | 26.3 | 26.3 | 26.3 |
| Fumed Silica | 1.0 | 1.0 | 1.0 | 1.0 |
| Sunflower oil | 0.5 | 0.5 | 0.5 | 0.5 |
| Fragrance | 0.8 | 0.8 | 0.8 | 0.8 |
| | C1 | C2 | C3 | C4 |
| **Hardness** (mm) | 15.4 17.1 (rerun) | 5.6 | 8.3 | 12 |

(continued)

| Centrifuge Results | | | | |
|---|---|---|---|---|
| % Oil Separation 25°C | 0.3 0.1 | None | 0.2 | 0.3 |
| % Oil Separation 45°C | 3.1 | 0.8 | 4.9 | 4.1 |
| % Oil Separation 50°C | 2.8 | 7.2 | 7.1 | 4.6 |
| * DC 9040 Silicone Elastomer from Dow Corning, a mixture of silicone elastomer in cyclomethicone. | | | | |

**Table 3**

| **Ingredient (% by weight)** | C5 | C6 | C7 | Example 6 |
|---|---|---|---|---|
| Cyclopentasiloxane | Balance to 100 | Balance to 100 | Balance to 100 | Balance to 100 |
| Paraffin Wax (MP 60-62°C) | 3.5 | 3.5 | | 3.5 |
| Microcrystalline Wax (MP 80/87°C) | | | 3.5 | |
| Polyethylene 400 (MP 84-86 °C) | | | | 2.0 |
| Petrolatum (MP 58-64 °C) | | | 3.5 | |
| Syncrowax® HGL-C C18-36 Acid Triglyceride (MP 70 °C) | | | | |
| Castor Wax (MP 84 °C) | | | | |
| Tribehenin (MP 83°C) | | | | |
| Stearyl Alcohol (MP 60°C) | 3.5 | | | |
| 12-Hydroxystearic acid (MP 72-77°C) | | 3.5 | | |
| Dimethicone (350 cst) | 10.0 | 10.0 | 10.0 | 10.0 |
| Silicone Elastomer* | 4.0 | 4.0 | 4.0 | 4.0 |
| Butylated Hydroxy Toluene (BHT) | 0.05 | 0.05 | 0.05 | 0.05 |
| Aluminum Zirconium Tetrachlorohydrex Glycine | 26.3 | 26.3 | 26.3 | 26.3 |
| Fumed Silica | 1.0 | 1.0 | 1.0 | 1.0 |
| Sunflower oil | 0.5 | 0.5 | 0.5 | 0.5 |
| Fragrance | 0.8 | 0.8 | 0.8 | 0.8 |
| | C5 | C6 | C7 | C8 |
| **Hardness** (mm) | 10.9 | 9.3 | 26.7 | 9.1 |
| **Centrifuge Results** | | | | |
| % Oil Separation 25°C | 0.1 | 1.8 | 0.9 | <0.1 |
| % Oil Separation 45°C | 4.6 | | 3.1 | 0.6 |
| % Oil Separation 50°C | 17.6 | | 11.1 | 5.9 |
| * DC 9040 Silicone Elastomer from Dow Corning, a mixture of silicone elastomer in cyclomethicone. | | | | |

Examples 1 to 5 had excellent stability at both room temperatures and 45°C. Example 6 had excellent stability at room temperature and good stability at 45°C. At 50°C, Examples 2 and 4 had better stability than Examples 1, 3, 5 and 6.

The C1 and C2 compositions were found to be gritty. The C6 and C7 compositions had significantly lower stability at room temperatures than the compositions of Examples 1 to 6, with the C7 composition being so soft that only about half as much sample as specified by the test could be extracted into the centrifuge tubes. Examples 1 to 6 exhibited significantly greater stability at 45°C than the C1, C3 to C5, and C7 compositions. The room temperature performance

of the C6 composition was so poor that it was not evaluated at higher temperatures. Examples 1 to 5 had significantly greater stability at 45°C than the C2 composition. Examples 1 to 6 all had a creamy, smooth texture.

## Claims

**1.** An antiperspirant composition comprising:

a) wax structurant comprising, based on the total weight of the wax structurant:

(i) from 50% by weight to 75% by weight of paraffin wax;
(ii) from 15% to 50% by weight of hydrocarbon wax comprising a microcrystalline wax other than paraffin wax, the melting point of the hydrocarbon wax other than paraffin wax being
at least 70°C and not more than 95°C, preferably not more than 90°C;
(iii) optionally, up to 10% by weight of silicone wax;

b) carrier oil;
c) at least 15% by weight, based on the total weight of the composition, of astringent antiperspirant active; and
d) optionally, silicone elastomer

wherein:

A) the composition is in the form of a substantially anhydrous soft solid;
B) the ratio, by weight, of the wax structurant to the carrier oil is from 0.085:1 to 0.2:1; and
C) carrier oil is present in an amount of at least 45% by weight, based on the total weight of the composition; and
D) additional wax, if present, does not exceed 20% by weight of the total weight of the wax structurant

**2.** A composition according to claim 1 which is free of organic ester wax.

**3.** A composition according to claim 1 or claim 2 which is free of fatty alcohol wax.

**4.** The composition according to any preceding claim wherein the wax structurant consists of paraffin wax and a hydrocarbon wax other than paraffin wax.

**5.** The composition according to any preceding claim that further comprises up to 1% by weight, based on the total weight of the composition, of silicone elastomer.

**6.** The composition according to any preceding claim that further comprises inorganic particulate thickener.

**7.** The composition according to any preceding claim wherein the inorganic particulate thickener comprises silica.

**8.** The composition according to any preceding claim wherein the carrier oil comprises from 50 to 85% by weight, based on the total weight thereof of volatile silicone oil.

**9.** The composition according to any preceding claim wherein the carrier oil further comprises non-volatile oil.

**10.** The composition according to claim 9 wherein the non-volatile oil comprises non-volatile silicone oil.

**11.** The composition according to claim 8 wherein the volatile oil comprises cyclomethicone.

**12.** The composition according to claim 8 wherein the volatile oil comprises cyclopentasiloxane.

## Patentansprüche

**1.** Schweißhemmende Zusammensetzung, umfassend:

a) Wachs-Strukturierungsmittel, umfassend, bezogen auf das Gesamtgewicht des Wachs-Strukturierungsmittels:

(i) von 50 Gewichts-% bis 75 Gewichts-% Paraffinwachs,
(ii) von 15 bis 50 Gewichts-% Kohlenwasserstoffwachs, umfassend ein mikrokristallines Wachs, verschieden vom Paraffinwachs,
wobei der Schmelzpunkt des Kohlenwasserstoffwachses, verschieden vom Paraffinwachs, mindestens 70°C und nicht mehr als 95°C, vorzugsweise nicht mehr als 90°, beträgt,
(iii) optional bis zu 10 Gewichts-% Siliconwachs,

b) Trägeröl,
c) mindestens 15 Gewichts-%, bezogen auf das Gesamtgewicht der Zusammensetzung, adstringierenden schweißhemmenden Wirkstoff und
d) optional Silicon-Elastomer,

wobei:

A) die Zusammensetzung in Form eines im Wesentlichen wasserfreien weichen Feststoffs vorliegt,
B) das Gewichtsverhältnis des Wachs-Strukturierungsmittels zu dem Trägeröl von 0,085:1 bis 0,2:1 beträgt und
C) das Trägeröl in einer Menge von mindestens 45 Gewichts-%, bezogen auf das Gesamtgewicht der Zusammensetzung, vorliegt und
D) zusätzliches Wachs, wenn vorhanden, 20 Gewichts-% des Gesamtgewichts des Wachs-Strukturierungsmittels nicht überschreitet.

**2.** Zusammensetzung nach Anspruch 1, die frei von organischem Esterwachs ist.

**3.** Zusammensetzung nach Anspruch 1 oder Anspruch 2, welche frei von Fettalkohol-Wachs ist.

**4.** Zusammensetzung nach irgendeinem vorhergehenden Anspruch, wobei das Wachs-Strukturierungsmittel aus Paraffinwachs und einem Kohlenwasserstoffwachs, verschieden vom Paraffinwachs, besteht.

**5.** Zusammensetzung nach irgendeinem vorhergehenden Anspruch, die ferner bis zu 1 Gewichts-%, bezogen auf das Gesamtgewicht der Zusammensetzung, Silikon-Elastomer umfasst.

**6.** Zusammensetzung nach irgendeinem vorhergehenden Anspruch, die ferner anorganisches teilchenförmiges Verdickungsmittel umfasst.

**7.** Zusammensetzung nach irgendeinem vorhergehenden Anspruch, wobei das anorganische teilchenförmige Verdickungsmittel Siliciumdioxid umfasst.

**8.** Zusammensetzung nach irgendeinem vorhergehenden Anspruch, wobei das Trägeröl von 50 bis 85 Gewichts-%, bezogen auf das Gesamtgewicht davon, flüchtiges Silikonöl umfasst.

**9.** Zusammensetzung nach irgendeinem vorhergehenden Anspruch, wobei das Trägeröl ferner nicht-flüchtiges Öl umfasst.

**10.** Zusammensetzung nach Anspruch 9, wobei das nicht-flüchtige Öl nicht-flüchtiges Siliconöl umfasst.

**11.** Zusammensetzung nach Anspruch 8, wobei das flüchtige Öl Cyclomethicon umfasst.

**12.** Zusammensetzung nach Anspruch 8, wobei das flüchtige Öl Cyclopentasiloxan umfasst.

## Revendications

**1.** Composition d’antiperspirant comprenant :

a) un structurant de cire comprenant, rapporté à la masse totale du structurant de cire :

(i) de 50 % en masse à 75 % en masse de cire de paraffine ;
(ii) de 15 % à 50 % en masse de cire hydrocarbonée comprenant une cire microcristalline différente de la cire de paraffine,
le point de fusion de la cire hydrocarbonée différente de la cire de paraffine étant d'au moins 70°C et d'au plus 95°C, de préférence d'au plus 90°C ;
(iii) éventuellement, jusqu'à 10 % en masse de cire de silicone ;

b) une huile de support ;
c) au moins 15 % en masse, rapporté à la masse totale de la composition, d'actif d'antiperspirant astringent ; et
d) éventuellement, un élastomère de silicone

dans laquelle :

A) la composition est dans la forme d'un solide mou essentiellement anhydre ;
B) le rapport, en masse, du structurant de cire à l'huile de support est de 0,085:1 à 0,2:1 ; et
C) l'huile de support est présente dans une quantité d'au moins 45 % en masse, rapporté à la masse totale de la composition ; et
D) une cire supplémentaire, si présente, n'excède pas 20 % en masse de la masse totale du structurant de cire.

**2.** Composition selon la revendication 1 laquelle est exempte de cire d'ester organique.

**3.** Composition selon la revendication 1 ou la revendication 2 laquelle est exempte de cire d'alcool gras.

**4.** Composition selon l'une quelconque des revendications précédentes, dans laquelle le structurant de cire consiste en cire de paraffine et en une cire hydrocarbonée différente de la cire de paraffine.

**5.** Composition selon l'une quelconque des revendications précédentes qui comprend de plus jusqu'à 1 % en masse, rapporté à la masse totale de la composition, d'élastomère de silicone.

**6.** Composition selon l'une quelconque des revendications précédentes qui comprend de plus un épaississant particulaire inorganique.

**7.** Composition selon l'une quelconque des revendications précédentes dans laquelle l'épaississant particulaire inorganique comprend de la silice.

**8.** Composition selon l'une quelconque des revendications précédentes, dans laquelle l'huile de support comprend de 50 à 85 % en masse, rapporté à la masse totale de celle-ci d'huile de silicone volatile.

**9.** Composition selon l'une quelconque des revendications précédentes, dans laquelle l'huile de support comprend de plus une huile non volatile.

**10.** Composition selon la revendication 9 dans laquelle l'huile non volatile comprend une huile de silicone non-volatile.

**11.** Composition selon la revendication 8 dans laquelle l'huile volatile comprend de la cyclométhicone.

**12.** Composition selon la revendication 8 dans laquelle l'huile de silicone comprend du cyclopentasiloxane.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2221039 A1
- US 3792068 A, Luedders
- GB 2299506 A
- US 5654362 A


### Non-patent literature cited in the description


- CRC Handbook of Chemistry and Physics. CRC Press